Europäisches Patentamt
European Patent Office
Office européen des brevets

(19)

(11) Veröffentlichungsnummer: **0 018 943**
**A1**

(12)

# EUROPÄISCHE PATENTANMELDUNG

(21) Anmeldenummer: **80810126.5**

(22) Anmeldetag: **15.04.80**

(51) Int. Cl.[3]: **C 07 D 249/08**
A 01 N 43/64
//C07C119/00

(30) Priorität: **20.04.79 CH 3749/79**

(43) Veröffentlichungstag der Anmeldung:
**12.11.80 Patentblatt 80/23**

(84) Benannte Vertragsstaaten:
**BE CH DE FR GB IT LI NL**

(71) Anmelder: **CIBA-GEIGY AG**
**Patentabteilung Postfach**
**CH-4002 Basel(CH)**

(72) Erfinder: **Gloor, Bernard, Dr.**
**Höhenweg 10**
**CH-4133 Pratteln(CH)**

(72) Erfinder: **Vogel, Christian, Dr.**
**Leimgrubenweg 23**
**CH-4102 Binningen(CH)**

(54) **1-Triazolo-N-(phenyl)-azomethin-Derivate, Verfahren zu ihrer Herstellung, diese Verbindungen enthaltende Mittel und ihre Verwendung in der Schädlingsbekämpfung.**

(57) Verbindungen der Formel I

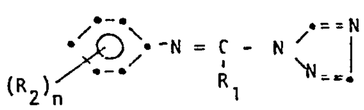

(I)

worin n für eine ganze Zahl zwischen 0 und 4 steht, $R_1$ $C_1$-$C_6$-Halogenalkyl, $C_3$-$C_6$ Cycloalkyl oder $C_3$-$C_6$ Halogencycloalkyl bedeutet, und $R_2$ eine durch n bestimmte Zahl gleicher oder verschiedener Substituenten aus der Gruppe Halogen, $C_1$–$C_4$-Alkyl, $C_2$-$C_4$-Alkoxy, Nitro, Trifluormethyl oder Cyano bedeutet.

Die Verbindungen sind für die Schädlingsbekämpfung geeignet und besitzen mikrobizide und akarizide Wirkung. Ferner sind sie als Synergisten zusammen mit bekannten Insektiziden, insbesondere insektiziden Phsophorsäureestern, wirksam.

EP 0 018 943 A1

BEZEICHNUNG GEÄNDERT
siehe Titelseite

5-12 326/1+2

1-Triazolo-N-(phenyl)-azomethin-Derivate

Die vorliegende Erfindung betrifft neue Verbindungen der Formel I

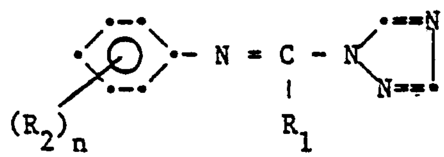

(I)

worin n für eine ganze Zahl zwischen 0 und 4 steht, $R_1$ $C_1$-$C_6$-Halogenalkyl, $C_3$-$C_6$ Cycloalkyl oder $C_3$-$C_6$ Halogencycloalkyl bedeutet, und $R_2$ eine durch n bestimmte Zahl gleicher oder verschiedener Substituenten aus der Gruppe Halogen, $C_1$-$C_4$-Alkyl, $C_1$-$C_4$-Alkoxy, Nitro, Trifluormethyl oder Cyano bedeutet.

Die Erfindung betrifft ferner Schädlingsbekämpfungsmittel, die Verbindungen der Formel I als Wirkstoffe enthalten, Verfahren zur Herstellung dieser Verbindungen und deren Verwendung in der Schädlingsbekämpfung.

Wegen ihrer Wirkung bevorzugt sind erfindungsgemäss Verbindungen der Formel I, die dadurch gekennzeichnet sind, dass

$R_1$ durch 1 bis 3 Halogenatome substituiertes Methyl, $C_3$-$C_6$-Cycloalkyl oder durch 1 Chloratom substituiertes $C_3$-$C_6$-Cycloalkyl und

$R_2$ Fluor, Chlor, Brom, Methoxy, Methyl, Aethyl oder Trifluormethyl bedeuten.

Hervorzuheben sind auch diejenigen Verbindungen der Formel I, worin

$R_1$ $C_3$-$C_6$-Cycloalkyl oder durch 1 Chloratom substutiertes Cyclohexyl und

$R_2$ Fluor, Chlor, Methoxy oder Trifluormethyl bedeuten;

sowie diejenigen Verbindungen der Formel I, worin

$R_1$ $C_3$-$C_6$-Cycloalkyl und

$R_2$ Chlor bedeuten.

Eine bevorzugte Gruppe von Verbindungen der Formel I ist dadurch gekennzeichnet, dass n für eine ganze Zahl zwischen 0 und 2, vorzugsweise für 1 oder 2, steht.

Besonders wertvoll sind erfindungsgemässe Verbindungen der Formel I, worin

n für 2 steht,

$R_1$ Cyclopropyl und

$R_2$ Chlor bedeuten.

Unter Alkyl oder als Alkylteil eines anderen Substituenten sind je nach Zahl der angegebenen C-Atome folgende Gruppen zu verstehen: Methyl, Aethyl, Propyl, Butyl, Pentyl oder Hexyl sowie ihre Isomere, wie z.B. iso-Propyl, iso-Butyl, sek.-Butyl, tert.-Butyl, iso-Pentyl usw.

Als Cycloalkyl sind Cyclopropyl, Cyclobutyl, Cyclopentyl und Cyclohexyl zu verstehen.

Halogen steht für Fluor, Chlor, Brom oder Jod.

Die Verbindungen der Formel I können in an sich bekannter Weise hergestellt werden, indem man eine Verbindung der Formel II

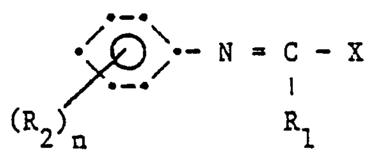

(II)

mit einer Verbindung der Formel III

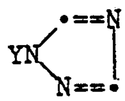

(III)

umsetzt, wobei in den Formeln II und III $R_1$, $R_2$ und n die für Formel I gegebenen Bedeutungen haben, X für ein Nukleofug und Y für Wasserstoff, ein Metallatom oder ein Ammoniumion stehen.

Als Nukleofug kommen in erster Linie der Rest einer anorganischen Säure wie z.B. Halogenid, vorzugsweise Chlorid oder Bromid, oder der einer organischen Sulfonsäure wie Tosyloxy oder Mesyloxy in Frage. Y als Metallatom ist vorteilhafterweise das Atom eines Alkali- oder Erdalkalimetalls wie z.B. Natrium oder Kalium und als Ammoniumion darf es auch substituiert sein. Das Verfahren zur Herstellung der Verbindungen der Formel I bildet ebenfalls einen Teil der Erfindung.

Umsetzungen werden in An- oder Abwesenheit von gegenüber den Reaktionsteilnehmern inerten Lösungsmitteln durchgeführt. Beispiele für geeignete Lösungsmittel sind: aliphatische oder aromatische Kohlenwasserstoffe wie Hexan, Cyclohexan, Benzol, Toluol, Xylol, Petroläther; halogenierte Kohlenwasserstoffe wie Chlorbenzol, Methylenchlorid, Aethylenchlorid, Chloroform, Trichloräthan; Aether oder ätherartige Verbindungen wie Dialkyläther, Tetrahydrofuran, Dioxan; Ketone wie Aceton, Methyläthylketon; Nitrile wie Acetonitril; N,N-Dialkylierte Amide wie Dimethylformamid sowie Gemische solcher Lösungsmittel.

Wenn Y=Wasserstoff ist, wird das Verfahren in Gegenwart eines säurebindenden Mittels durchgeführt. Als säurebindende Mittel kommen beispielsweise folgende Substanzen in Betracht: Basen wie Hydroxide und Carbonate von Alkali- und Erdalkalimetallen wie z.B. Soda,

Pottasche, Aetznatron, Aetzkali; tertiäre Amine wie Triäthylamin, Dimethylanilin, Pyridinbasen; Hydride wie Natrium oder Calciumhydrid, Amide wie Natrium- oder Kaliumamid, Lithiumdiisopropylamid oder aber ein Ueberschuss des 1,2,4-Triazols der Formel III.

Die Reaktionstemperaturen liegen normalerweise zwischen -20° und 120° Celsius vorzugsweise -10° bis 100° Celsius.

Die Ausgangsstoffe der Formel II werden nach an sich bekannten Methoden hergestellt wie z.B. nachfolgend schematisch dargestellt

a) 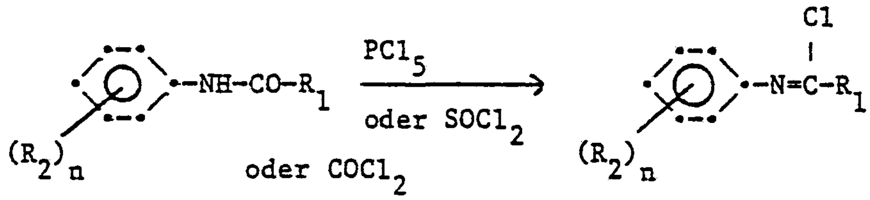

(II)

(Houben-Weyl "Methoden der organ. Chemie" Bd. 8, 673)

b) Herstellung von II in situ und Weiterreaktion mit III

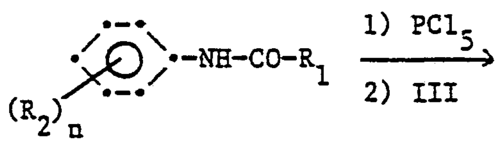

I

(vgl. Kaufmann et al. Chem.Ber. 75, 1585 (1942))

oder 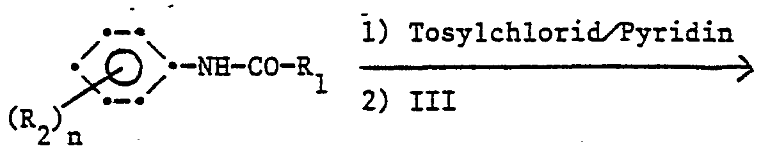

I

(vgl. Oxley et al. JCS, 1948, 1618)

Die Ausgangsstoffe der Formel II sind zum Teil neu und besitzen eine gewisse fungizide Wirkung. Sie gehören ebenfalls zur Erfindung, desgleichen Schädlingsbekämpfungsmittel, die Verbindungen der Formel II als Wirkstoffe enthalten.

Die Verbindungen der Formel I können für sich allein oder zusammen mit geeigneten Trägern und/oder anderen Zuschlagstoffen verwendet werden. Geeignete Träger und Zuschlagstoffe können fest oder flüssig sein und entsprechen den in der Formulierungstechnik üblichen Stoffen wie z.B. natürlichen oder regenerierten mineralischen Stoffen, Lösungs-, Dispergier-, Netz-, Haft-, Verdickungs-, Binde- oder Düngemitteln. Die Herstellung solcher Mittel erfolgt in an sich bekannter Weise durch inniges Vermischen und Vermahlen der Bestandteile. Zur Applikation können die Verbindungen der Formel I in den folgenden Aufarbeitungsformen vorliegen (wobei die Gewichtsprozentangaben vorteilhafte Mengen an Wirkstoff darstellen).

Feste Aufarbeitungsformen: Stäubemittel, Streumittel, (bis 10 %) Granulate, Umhüllungsgranulate, Imprägnierungsgranulate und Imprägnierungsgranulate und Homogengranulate, Pellets (Körner) (1 bis 80 %)

Flüssige Aufarbeitsformen:

a) in Wasser dispergierbare Wirkstoffkonzentrate: Spritzpulver (wettable powders) Pasten, 25-90 % in der Handelspackung, 0,01 bis 15 % in gebrauchsfertiger Lösung, Emulsionen, Lösungskonzentrate (10 bis 50 %); (0,01 bis 15 % in gebrauchsfertiger Lösung)

b) Lösungen: Aerosole:

Der Gehalt an Wirkstoff in den oben beschriebenen Mitteln liegt zwischen 0,1 und 95 Gewichtsprozent. Solche Mittel sind gleichfalls Gegenstand dieser Erfindung.

Die Verbindungen der Formel I besitzen ein für die praktischen Bedürfnisse sehr günstiges Mikrobizid-Spektrum zum Schutz von Kulturpflanzen, ohne diese durch unerwünschte Nebenwirkungen nachteilig zu beeinflussen. Kulturpflanzen seien im Rahmen vorliegender Erfindung beispielsweise Getreide, Mais, Reis, Gemüse, Zuckerrüben, Soja, Erdnüsse, Obstbäume, Zierpflanzen, Reben, Hopfen, Gurkengewächse (Gurken,

Kürbis, Melonen), Solanaceen wie Kartoffeln, Tabak und Tomaten, sowie auch Bananen-, Kakao- und Naturkautschuk-Gewächse.

Mit den Wirkstoffen der Formel I können an Pflanzen oder Pflanzenteilen (Früchte, Blüten, Laubwerk, Stengel, Knollen, Wurzeln) dieser und verwandter Nutzkulturen die auftretenden Mikroorganismen eingedämmt oder vernichtet werden, wobei auch später zuwachsende Pflanzenteile von derartigen Pilzen verschont bleiben. Die Wirkstoffe sind gegen die den folgenden Klassen angehörenden phytopathogenen Pilzen wirksam: Ascomycetes (z.B. Erysiphaceae, Fusarium, Podosphaera, Venturia, Helminthosporium); Basidiomycetes wie vor allem Rostpilze (z.B. Puccinia, Tilletia, Rhizoctonia); Fungi imperfecti und die der Klasse Phycomycetes angehörenden Oomycetes wie Phytophthora und Plasmopara. Die Wirkstoffe sind auch gegen pflanzenschädigende Akarina aktiv. Als Akarina können z.B. Tetranychus urticae und Tetranychus cinnabarius genannt werden, die in allen Stadien (Ei, Larven, Adulte) mit Verbindungen der Formel I bekämpft werden können. Diese Aktivität wird hauptsächlich bei solchen Verbindungen der Formel I angetroffen, worin $R_1$ für fluorsubstituiertes Alkyl steht, das auch durch weiteres Halogen substituiert sein kann. Bevorzugt wird allerdings Alkyl das ausschliesslich durch Fluor substituiert ist.

Die Verbindungen sind ferner gegen phytopathogene Bakterien wie Xanthomonas sp., Pseudomonas sp., Erwinia sowie Corynebacterium aktiv.

Die Erfindung betrifft somit ferner die Verwendung der Verbindungen der Formel I zur Bekämpfung von phytopathogenen Mikroorganismen und Vertretern der Ordnung Akarina. Als Einzelverbindung wird hierfür z.B. 1-[1-(2,4-Dichlorphenylimino)-2-dichloräthyl]-1H-1,2,4-triazol bevorzugt.

Die Verbindungen der Formel I können, um sie an gegebene Umständen anzupassen, zur Erhöhung der Wirksamkeit und Verbreiterung des Wirkungsspektrums zusammen mit anderen geeigneten Pestiziden, wie z.B.

Fungiziden, Bakteriziden, Insektiziden, Akariziden, Herbiziden oder den Pflanzenwuchs beeinflussenden Wirkstoffen eingesetzt werden. So sind die erfindungsgemässen Verbindungen der Formel I auch als Synergisten in Kombination mit anderen bekannten Insektiziden vom Phosphorsäureester-, Chlorkohlenwasserstoff-, Carbamat- und Pyrethroid-Typ, insbesondere gegenüber resistenten Insektenstämmen, wirksam. Ueberraschenderweise ist die insektizide Wirksamkeit der genannten Wirkstoffkombinationen wesentlich höher als die Summe der Wirkungen der einzelnen Wirkstoffe. Es liegt demnach ein echter synergistischer Effekt vor und nicht nur eine Wirkungsergänzung oder -Summierung. Dieser synergistische Effekt tritt besonders stark bei bestimmten Konzentrationsverhältnissen hervor. Im Rahmen der vorliegenden Erfindung hat sich ein Gew.-Verhältnis von bekanntem Wirkstoff zu einer synergistisch wirksamen Verbindung der Formel I von 9:1 bis 1:9, vorzugsweise 4:1 bis 1:4, als günstig erwiesen. Die erfindungsgemässen synergistischen Wirkstoffkombinationen zeichnen sich dadurch aus, dass schon bei geringer Dosierung der angestrebte Effekt eintritt, was vom Standpunkt der Toxikologie und Oekologie einen erheblichen Vorteil darstellt. Von besonderem Interesse erweist sich die Anwendung der erfindungsgemässen synergistischen Wirkstoffkombinationen bei der Bekämpfung resistenter und auch multiresistenter Insektenstämme. Es handelt sich hierbei in der Hauptsache um Resistenz gegenüber Phosphorsäureester. Derartige resistente Stämme konnten mit den bisher üblichen Insektiziden nur schwierig, bzw. nur unter Einsatz bedenklich hoher Wirkstoffdosierungen bekämpft werden.

Unter den Verbindungen der Formel I hat sich das 1-(2,4-Dichlorphenylimino-cyclopropylmethyl)-1H-1,2,4-triazol als besonders wirksamer Synergist erwiesen.

Die erfindungsgemässen synergistischen Wirkstoffkombinationen weisen bei guter Pflanzenverträglichkeit und geringer Warmblütertoxizität ausgezeichnete Wirksamkeit als Schädlingsbekämpfungsmittel, vor allem als Kontaktinsektizide zur Bekämpfung von Pflanzen und Tiere befallenden Insekten auf.

Die Verbindungen der Formel I enthaltenden synergistischen Wirkstoffkombinationen sind wirksam zur Bekämpfung von Insekten der Familien: Acrididae, Blattidae, Gryllidae,Gryllotalpidae, Tettigoniidae, Cimicidae, Phyrrhocoridae, Reduviidae, Aphididae, Delphacidae, Diaspididae, Pseudococcidae, Chrysomelidae, Coccinellidae, Bruchidae, Scarabaeidae, Dermestidae, Tenebrionidae, Curculionidae, Tineidae, Noctuidae, Lymantriidae, Pyralidae, Galleridae, Culicidae, Tipulidae, Stomoxydae, Muscidae, Calliphoridae, Trypetidae und Pulicidae.

Neben ihrer sehr günstigen Wirkung gegenüber Fliegen, wie z.B. Musca domestica, und Mückenlarven eignen sich die erfindungsgemässen synergistischen Wirkstoffkombinationen insbesondere zur Bekämpfung von pflanzenschädigenden Frassinsekten in Zier- und Nutzpflanzungen, insbesondere in Baumwollkulturen (z.B. Spodoptera littoralis) sowie in Gemüsekulturen (z.B. Myzus persicae).

Die erfindungsgemässen synergistischen Wirkstoffkombinationen sind weiterhin zur Bekämpfung von Ektoparasiten an Haus- und Nutztieren, z.B.,durch Tier-, Stall- und Weideehandlung geeignet.

Die nachfolgenden Beispiele dienen zur näheren Erläuterung der Erfindung. Die Temperaturangaben beziehen sich auf Celsiusgrade, Druckangaben beziehen sich auf Millibar und Teile auf Gewichtsteile.

Herstellungsbeispiele

Beispiel 1: 1-[1-(2,4-Dichlorphenylimino)-2-fluor-äthyl]-1H-1,2,4-triazol der Formel

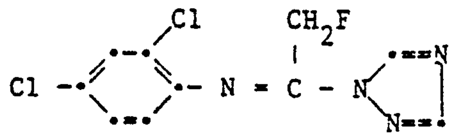

a) Ausgangsmaterial

Die Lösung von 324 g (2,0 Mol) 2,4-Dichloranilin in 1,3 l Chlorbenzol wurde auf 100-110° aufgeheizt und bei dieser Temperatur innerhalb 1 1/2 Stunden unter Rühren mit 158 ml (2,2 Mol) Fluoracetylchlorid tropfweise versetzt. Nach Beendigung der HCl-Entwicklung wurde auf Raumtemperatur abgekühlt, zur Reaktionsmischung 416 g (2,0 Mol) Phosphorpentachlorid hinzugefügt, erneut innerhalb 1 Stunde auf 110° aufgeheizt und 2 Stunden bei dieser Temperatur weitergerührt. Das Lösungsmittel und das entstandene Phosphoroxichlorid wurden hierauf am Wasserstrahlvacuum abgedampft und der Rückstand im Hochvakuum destilliert. Es wurden 463 g N-(1-Chlor-2-fluor-äthyliden)-2,4-dichloranilin als farblose Flüssigkeit, Sdp: 64-66°/$5 \cdot 10^{-3}$ erhalten.

Auf analoge Weise oder nach einer der hierin beschriebenen Methoden wurden folgende Zwischenprodukte der Formel II hergestellt.

(X = Cl)

| $R_1$ | $(R_2)_n$ | physikalische Konstante |
|---|---|---|
| $-CHCl_2$ | 2,4-$(Cl)_2$ | Kp 90-94° / 0,001 |
| $-CH_2Cl$ | 2,4-$(Cl)_2$ | Kp 90-92° / 0,001 |
| $-CCl_3$ | 2,4-$(Cl)_2$ | Kp 107-111° / 0,01 |
| $-CHCl_2$ | 2,5-$(Cl)_2$ | Kp 96-99° / 0,001 |
| $-CH_2F$ | 2,4-$(Cl)_2$ | Kp 64-66° / 0,005 |
| $-CHClF$ | 2,4-$(Cl)_2$ | Kp 69-72° / 0,001 |
| $-CCl_2F$ | 2,4-$(Cl)_2$ | Kp 76-79° / 0,01 |
| $-CHCl_2$ | - (n=0) | Kp 75-78° / 0,3 |
| $-CCl_3$ | - (n=0) | Kp 136-138° / 16 |
| 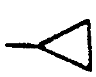 | - (n=0) | |

(X = Cl)

| $R_1$ | $(R_2)_n$ | physikalische Konstante |
|---|---|---|
| 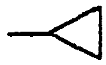 | 4-Br | Kp 98-103° / 0,1 |
| 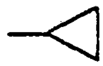 | 2,4-$(Cl)_2$ | Kp 100-105° / 0,3 |
| 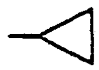 | 3,4-$(Cl)_2$ | Kp 130-135° / 0,1 |
| 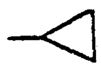 | 4-$OCH_3$ | |
| 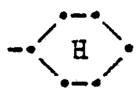 | 4-Br | Kp 132-133° / 0,4 |
| 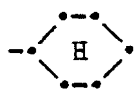 | 3,4-$(Cl)_2$ | Kp 134-140 / 0,15 |
| 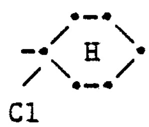 | 4-Br | |
| 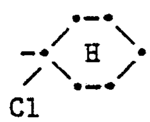 | 3,4$(Cl)_2$ | Kp 158-164° / 0,4 |
| $-CHCl_2$ | 2-Chlor,-6-Methyl | Kp 84-88° / 0,005 |
| $-CHCl_2$ | 3,5-$(Cl)_2$ | Kp 104-108° / 0,005 |
| $-CHCl_2$ | 2,6-$(Cl)_2$ | Kp 92-94° / 0,005 |
| $-CH(Cl)(CH_3)_2$ | 4-Br | Kp 93-95° / 0,2 |
| $-CH(Cl)(CH_3)_2$ | 2,4-$(Cl)_2$ | Kp 72-75° / 0,15 |
| $-CH(Cl)(CH_3)_2$ | 3,4-$(Cl)_2$ | Kp 99-101° / 0,1 |
| $-CH(Cl)(CH_3)_2$ | 4-$OCH_3$ | Kp 93-94° / 0,15 |
| $-CH_2Cl$ | 2,6-$(CH_3)_2$ | Kp 82-83° / 1 |
| $-CHCl_2$ | 2,6-$(CH_3)_2$ | Kp 72-76° / 0,005 |
| $-CCl_3$ | 2,6-$(CH_3)_2$ | Kp 82-85° / 0,03 |
| $-CCl_3$ | 2-$CH_3$, 6-$C_2H_5$ | Kp 94-98° / 0,01 |

b) Endprodukt

Unter Rühren und Kühlung wurden bei Raumtemperatur 10 g (0,11 Mol) 1,2,4-Triazol-natriumsalz portionenweise zu der Lösung von 24 g (0,1 Mol) N-(1-Chlor-2-fluor-äthyliden)-2,4-dichloranilin in 150 ml Dimethylformamid hinzugefügt. Nach der Zugabe wurde die Reaktionsmischung noch 1 Stunde bei Raumtemperatur gerührt und hierauf, ebenfalls unter Eiskühlung, mit 150 ml Wasser versetzt. Der ausgefallene Niederschlag wurde abfiltriert, mit Wasser gewaschen und aus 200 ml Isopropyl-äther umkristallisiert. Die Ausbeute am Endprodukt betrug 18 g (hellgelbe Kristalle), Smp. 100-102°.

Auf analoge Weise oder nach einer der hierin beschriebenen Methoden können folgende Verbindungen der Formel I hergestellt werden.

| $R_1$ | $(R_2)_n$ | physikalische Daten |
|---|---|---|
| $-CHCl_2$ | 2,4-$(Cl)_2$ | Smp. 72-75°C |
| $-CH_2Cl$ | 2,4-$(Cl)_2$ | Smp. 75-77°C |
| $-CCl_3$ | 2,4-$(Cl)_2$ | Smp. 67-69°C |
| $-CHCl_2$ | 2,5-$(Cl)_2$ | Smp. 93-96°C |
| $-CH_2F$ | 2,4-$(Cl)_2$ | Smp. 100-102°C |
| -CHClF | 2,4-$(Cl)_2$ | Smp. 52-55°C |
| $-CCl_2F$ | 2,4-$(Cl)_2$ | Kp 109-114°C/0,005 Torr |
| $-CHCl_2$ | - (n=0) | Smp. 73-75°C |
| $-CCl_3$ | - (n=0) | Smp. 48-51°C |
| $-CHCl_2$ | 2-Cl, 5-$CH_3$ | Smp. 93-96°C |
| $-CHCl_2$ | 3,5-$(Cl)_2$ | Smp. 88-90°C |
| $-CHCl_2$ | 2,6-$(Cl)_2$ | Smp. 81-89°C |
| $-CHCl_2$ | 3,4-$(Cl)_2$ | Smp. 109-112°C |
| $-CCl(CH_3)_2$ | 2,4-$(Cl)_2$ | Smp. 54-56°C |
| $-CCl(CH_3)_2$ | 3,4-$(Cl)_2$ | Smp. 56-58°C |
| $-CCl(CH_3)_2$ | 4-$CH_3O$ | Smp. 103-105°C |

| $R_1$ | $(R_2)_n$ | physikalische Daten |
|---|---|---|
| $-CCl(CH_3)_2$ | 4-Br | Smp. 87-89°C |
| $-CCl(CH_3)_2$ | - (n=0) | Smp. 36-40°C |
| $-CCl(CH_3)_2$ | $4-CH_3$ | Smp. 67-70°C |
| $-CCl(CH_3)_2$ | $3-CF_3$, 4-Cl | Smp. 60-63°C |
| 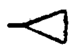 | - (n=0) | |
| 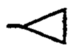 | 4-Br | Smp. 61-63°C |
| 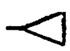 | $2,4-(Cl)_2$ | Oel; NMR-Spektrum:*)<br>δ = 1,05 m, 1,35 m(4H)<br>δ = 2,05 m (1H)<br>δ = 6,8 d,J=8,5 Hz(1H)<br>δ = 7,3 m (2H)<br>δ = 7,9 s (1H)<br>δ = 8,85 s (1H) |
| 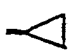 | $3,4-(Cl)_2$ | NMR-Spektrum*):<br>δ = 1,1 m,1,35 m (4H)<br>δ = 2,1 m (1H)<br>δ = 6,8 dd, J=8,0 Hz, J=2,5 Hz (1H)<br>δ = 7,05 d, J=2,5 Hz(1H)<br>δ = 7,43 d, J=8,0 Hz (1H)<br>δ = 8,0 s (1H)<br>δ = 8,93 s (1H) |
| 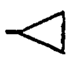 | $4-OCH_3$ | |

*) Chemische Verschiebung δ in ppm, relativ zu Tetramethylsilan; alle Aufnahmen bei 60 MHz; Lösungen in $CDCl_3$.

| $R_1$ | $(R_2)_n$ | physikalische Daten |
|---|---|---|
| 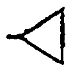 | 3-F, 4-Cl | NMR-Spektrum*):<br>δ= 1,15 m, 1,42 m (4H)<br>δ= 2,1 m (1H)<br>δ= 7,1 dd, J=8,5 Hz,<br>J=2 Hz(1H)<br>δ= 7,35 d, J=2 Hz(1H)<br>δ= 7,55 d, J=8,5 Hz(1H)<br>δ= 8,03 s (1H)<br>δ= 9,05 s (1H) |
| 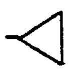 | 2-$CH_3$, 4-Cl | Smp. 53-55°C |
| 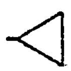 | 3-$CF_3$ | NMR-Spektrum *)<br>δ= 1,1 m, 1,35 m (4H)<br>δ= 2,1 m (1H)<br>δ= 7,15 m, 7,4 m (4H)<br>δ= 7,97 s (1H)<br>δ= 8,95 s (1H) |
| 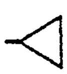 | 3,5-$(CF_3)_2$ | Smp. 82-84°C |
| 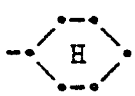 | 4-Br | |
| 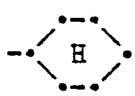 | 3,4-$(Cl)_2$ | |
| 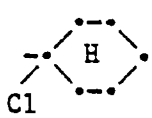 | 4-Br | |

*) Chemische Verschiebung δ in ppm, relativ zu Tetramethylsilan; alle Aufnahmen bei 60 MHz; Lösungen in $CDCl_3$.

| $R_1$ | $(R_2)_n$ | physikalische Daten |
|---|---|---|
| 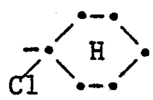 | $3,4-(Cl)_2$ | honigartig;<br>NMR-Spektrum*):<br>δ = 1,6 m, 2,1 m (11H)<br>δ = 6,37 dd, J=9 Hz, J=2 Hz(1H)<br>δ = 6,75 d, J=2 Hz(1H)<br>δ = 7,13 d, J=9 Hz(1H)<br>δ = 7,82 s (1H)<br>δ = 8,16 s (1H) |
| $-CHCl_2$ | 4-Cl | |
| $-CH_2F$ | 4-Cl | |
| $-CHCl_2$ | 4-Br | |
| $-CH_2F$ | 4-Br | |
| $-CHCl_2$ | $4-(CH_3)_3C-$ | |
| $-CHCl_2$ | $3-CF_3$, 4-Cl | |
| $-CHCl_2$ | $2-CH_3O$, 4-Cl | |
| $-CH_2F$ | $2-CH_3O$, 4-Cl | |
| $-CHCl_2$ | $2-CH_3$, 4-Cl | |
| $-CH_2F$ | $2-CH_3$, 4-Cl | |
| $-CCl_3$ | $3-NO_2$ | |
| $-CH_2Cl$ | $2,6-(CH_3)_2$ | Kp 82-83°C/4 Torr |
| $-CHCl_2$ | $2,6-(CH_3)_2$ | Smp. 97-98°C |
| $-CCl_3$ | $2,6-(CH_3)_2$ | Smp. 78-79°C |
| $-CCl_3$ | $2-CH_3$, $6-C_2H_5$ | Kp. 94-98°C/0,01 Torr. |

*) Chemische Verschiebung δ in ppm, relativ zu Tetramethylsilan; alle Aufnahmen bei 60 MHz; Lösungen in $CDCl_3$.

Formulierungsbeispiele

Beispiel 2:

Stäubemittel: Zur Herstellung eines a) 5 %igen, b) 2 %igen und c) 80 %igen Stäubemittels werden die folgenden Stoffe verwendet:

a) 5 Teile Wirkstoff
95 Teile Talkum;

b) 2 Teile Wirkstoff
1 Teil hochdisperse Kieselsäure,
97 Teile Talkum;

c) 80 Teile Wirkstoff
17 Teile Talkum
3 Teile hochdisperse Kieselsäure

Die Wirkstoffe werden mit den Trägerstoffen vermischt und vermahlen und können in dieser Form zur Anwendung verstäubt werden.

Beispiel 3:

Granulat: Zur Herstellung eines 5 %igen Granulates werden die folgenden Stoffe verwendet:

5 Teile Wirkstoff
0,25 Teile epoxydiertes Pflanzenöl,
0,25 Teile Cetylpolyglykoläther,
3,50 Teile Polyäthylenglykol
91 Teile Kaolin (Korngrösse 0,3 - 0,8 mm).

Die Aktivsubstanz wird mit dem epoxydierten Pflanzenöl vermischt und mit 6 Teilen Aceton gelöst, hierauf wird Polyäthylenglykol und Cetylpolyglykoläther zugesetzt. Die so erhaltene Lösung wird auf Kaolin aufgesprüht, und anschliessend wird das Aceton im Vakuum verdampft. Ein derartiges Mikrogranulat wird vorteilhaft zur Bekämpfung von Bodenpilzen verwendet.

Beispiel 4:

Spritzpulver: Zur Herstellung eines a) 70 %igen b) 40 %igen c) und d) 25 %igen e) 10 %igen Spritzpulvers werden folgende Bestandteile verwendet:

a) 70 Teile Wirkstoff
5 Teile Natriumdibutylnaphthylsulfonat,
3 Teile Naphthalinsulfonsäuren-Phenolsulfonsäuren-Formaldehyd-Kondensat 3:2:1,
10 Teile Kaolin
12 Teile Champagne-Kreide;

b) 40 Teile Wirkstoff
5 Teile Lingninsulfonsäure-Natriumsalz,
1 Teil Dibutylnaphthalinsulfonsäure-Natriumsalz,
54 Teile Kieselsäure;

c) 25 Teile Wirkstoff
4,5 Teile Calcium-Ligninsulfonat,
1,9 Teile Champagne-Kreide/Hydroxyäthylcellulose-Gemisch (1:1),
1,5 Teile Natrium-dibutyl-naphthalinsulfonat,
19,5 Teile Kieselsäure,
19,5 Teile Champagne-Kreide,
28,1 Teile Kaolin;

d) 25 Teile Wirkstoff
2,5 Teile Isooctylphenoxy-polyoxyäthylen-äthanol,
1,7 Teile Champagne-Kreide/Hydroxyäthylcellulose-Gemisch (1:1),
8,3 Teile Natriumaluminiumsilikat,
16,5 Teile Kieselgur,
46 Teile Kaolin;

e) 10 Teile Wirkstoff
3 Teile Gemisch der Natriumsalze von gesättigten Fettalkoholsulfaten,
5 Teile Naphthalinsulfonsäure/Formaldehyd-Kondensat,
82 Teile Kaolin;

Die Wirkstoffe werden in geeigneten Mischern mit den Zuschlagstoffen innig vermischt und auf entsprechenden Mühlen und Walzen vermahlen. Man erhält Spritzpulver von vorzüglicher Benetzbarkeit und Schwebefähigkeit, die sich mit Wasser zu Suspensionen der gewünschten Konzentration verdünnen und insbesondere zur Blattapplikation verwenden lassen.

Beispiel 5:

Emulgierbare Konzentrate: Zur Herstellung eines 25 %igen emulgierbaren Konzentrates werden folgende Stoffe verwendet:

25 Teile Wirkstoff
2,5 Teile epoxydiertes Pflanzenöl,
10 Teile eines Alkylarylsulfonat/Fettalkoholpolyglykoläther-Gemisches,
5 Teile Dimethylformamid,
57,5 Teile Xylol.

Aus solchen Konzentraten können durch Verdünnen mit Wasser Emulsionen der gewünschten Anwendungskonzentration hergestellt werden, die besonders zur Blattapplikation geeignet sind.

Biologische Beispiele

Beispiel 6:

Wirkung gegen Erysiphe graminis auf Gerste

Ca. 8 cm hohe Gerstenpflanzen wurden mit einer aus Spritzpulver des Wirkstoffes hergestellten Spritzbrühe (0,02 % Aktivsubstanz) besprüht. Nach 4 Stunden wurden die behandelten Pflanzen mit Konidien

des Pilzes bestäubt. Die infizierten Gerstenpflanzen wurden in einem Gewächshaus bei ca. 22°C aufgestellt und der Pilzbefall nach 10 Tagen beurteilt.

Beispiel 7:

Wirkung gegen Piccinia graminis auf Weizen

Weizenpflanzen wurden 6 Tage nach der Aussaat mit einer aus Spritzpulver des Wirkstoffes hergestellten Spritzbrühe (0,06 %) Aktivsubstanz) besprüht. Nach 24 Stunden wurden die behandelten Pflanzen mit einer Uredosporensuspension des Pilzes infiziert. Nach einer Inkubation während 48 Stunden bei 95-100 % relativer Luftfeuchtigkeit und ca. 20°C wurden die infizierten Pflanzen in einem Gewächshaus bei ca. 22°C aufgestellt.

Die Beurteilung der Rostpustelnentwicklung erfolgte 12 Tage nach der Infektion.

Beispiel 8:

Wirkung gegen Podosphaera leucotricha auf Aepfel

Apfelsämlinge im 5-Blattstadium wurden mit einer aus Spritzpulver des Wirkstoffes hergestellten Spritzbrühe (0,06 % Aktivsubstanz) besprüht.

Nach 24 Stunden wurden die behandelten Pflanzen mit einer Konidiensuspension des Pilzes infiziert und in einer Klimakammer bei 70 % relativer Luftfeuchtigkeit und 20°C aufgestellt. Die Beurteilung des Pilzbefalls erfolgte 12 Tage nach der Infektion.

Beispiel 9:

Wirkung gegen Venturia inaequalis auf Aepfel

Apfelsämlinge im 5 Blattstadium wurden mit einer aus Spritzpulver des Wirkstoffes hergestellten Spritzbrühe (0,06 % Aktivsubstanz) besprüht.

Nach 24 Stunden wurden die behandelten Pflanzen mit einer Konidiensuspension des Pilzes infiziert. Die Pflanzen wurden dann während 5 Tagen bei 90-100 % relativer Luftfeuchtigkeit inkubiert und während 10 weiteren Tagen in einem Gewächshaus bei 20-24°C aufgestellt. Der Schorfbefall wurde 15 Tage nach der Infektion beurteilt.

Beispiel 10:
Wirkung gegen Plasmopara viticola auf Reben

Im 4-5 Blattstadium wurden Rebensämlinge mit einer aus Spritzpulver des Wirkstoffes hergestellten Spritzbrühe (0,06 % Aktivsubstanz) besprüht. Nach 24 Stunden wurden die behandelten Pflanzen mit einer Sporangiensuspension des Pilzes infiziert. Nach einer Inkubation während 6 Tagen bei 95-100 % relativer Luftfeuchtigkeit und 20°C wurde der Pilzbefall beurteilt.

Beispiel 11:
Wirkung gegen Tilletia caries

Tilletiasporen werden in einer Spritzbrühe enthaltend 600 ppm Wirkstoff während 15 min. suspendiert. Das Sporen-Substanz-Gemisch wird tropfweise auf die Oberfläche von fein gesiebter, angefeuchteter Erde in Petrischalen pipettiert. Die so zubereiteten Erdschalen werden bei hoher Luftfeuchtigkeit und einer Temperatur von 20°C aufgestellt. Nach ca. 10 Tagen wird die Sporenkeimung unter der Lupe beurteilt. Die Wirkung der Testsubstanzen wird auf Grund der Anzahl und der Länge der Keimschläuche ermittelt.

Verbindungen der Formel I zeigten in den obigen Versuchen eine gute Wirkung.

Unter anderem zeigten folgende Verbindungen der Formel I bei folgenden Pilzen eine Hemmung des Befalls auf weniger als 20 % verglichen mit Kontrollpflanzen.

Bei Erysiphe graminis: 1

Bei Puccinia graminis: 1, 2, 4, 5, 8

Bei Helminthosporium gramineum: 2, 3, 6

Bei Podosphaera leucotricha: 1

Bei Venturia inaequalis: 1, 8

Bei Plasmopara viticola: 1, 8

Bei Tilletia caries: 2, 4

Beispiel 12:

Wirkung gegen Xanthomonas vesicatoria auf Paprika

a) Residual-protektive Wirkung

Paprikapflanzen der Sorte "California Wonder" wurden nach 3-wöchiger Anzucht im Gewächshaus mit der Prüfsubstanz in Form einer Spritzbrühe (0,06 % Aktivsubstanz) besprüht. Nach eintägigem Antrocknen dieses Spritzbelages wurden die Pflanzen in einem Klimaraum bei 26°C und 95-100 % relativer Luftfeuchtigkeit aufgestellt und durch Besprühen der Blattunterseiten mit einer standardisierten Suspension von Xanthomonas vesicatoria infiziert. Nach 6-tägiger Inkubation in demselben Raum entstanden auf den Blättern runde, anfangs wässrige, später nekrotische, aufgehellte Flecken. Das Ausmass dieser Flecken diente zur Beurteilung der residualen Wirksamkeit der Prüfsubstanz.

b) Systemische Wirkung

Paprikapflanzen der Sorte "California Wonder" wurden nach 3-wöchiger Anzucht im Gewächshaus mit einer Suspension der Prüfsubstanz begossen (0,006 % Aktivsubstanz bezogen auf das Erdvolumen). Drei Tage nach dieser Behandlung wurden die Pflanzen in einem Klimaraum bei 20°C und 95-100 % relativer Luftfeuchtigkeit aufgestellt und durch Besprühen der Blattunterseiten mit einer standardisierten Suspension von Xanthomonas vesicatoria infiziert. Nach 6-tägiger Inkubation in demselben Raum entstanden auf den Blättern runde, anfangs wässrige,später nektrotische, aufgehellte Flecken. Das Ausmass dieser Flecken diente zur Beurteilung der systemischen Wirksamkeit der Prüfsubstanz.

In den obigen Versuchen zeigten Verbindungen z.B. Nr. 3, 4, 5, 6 der Formel I eine starke bakterizide Wirkung.

Beispiel 13:

Akarizide Wirkung: Tetranychus urticae (OP-sensible) und Tetranychus cinnabarius (OP-tolerant)

Die Primärblätter von Phaseolus vulgaris Pflanzen wurden 16 Stunden vor dem Versuch auf akarizide Wirkung mit einem infestierten Blattstück aus einer Massenzucht von Tetranychus urticae (OP-sens) oder Tetranychus cinnabarius (OP-tol) belegt. (Die Toleranz bezieht sich auf die Verträglichkeit von Diazinon.) Die so behandelten, durch die übergelaufenen beweglichen Individuen infestierten Pflanzen wurden mit einer Versuchslösung enthaltend 400 , 200 oder 100 ppm der erfindungsgemässen Verbindung bis zur Tropfnässe besprüht.

Nach 24 Stunden und wiederum nach 7 Tagen wurde unter dem Binokular auf lebende und tote Individuen ausgewertet.

Man verwendete pro Konzentration und pro Testspezies eine Pflanze. Während des Versuchsverlaufs standen die Pflanzen in Gewächskabinen bei 25°C.

Unter anderem zeigten die Verbindungen 5 und 6 im obigen Versuch eine gute Wirkung gegen Adulte, Larven und Eier der Spezies Tetranychus urticae und Tetranychus cinnabarius.

Beispiel 14: Synergistische Insektizidwirkung auf resistente Stämme von Musca domestica und Spodoptera littoralis:

a) Es wurden adulte Stubenfliegen (Musca domestica), die gegen insektizide Phosphorsäureester Resistenz entwickelt hatten, durch Kälteschock inaktiviert. Dann wurde mittels eines Mikro-Applikators 1 µl der jeweiligen Wirkstofflösung (Lösungsmittel Aceton/Wasser : 80/20) auf die Oberseite des Abdomens eines jeden Versuchstieres aufgebracht und die prozentuale Mortalität der Fliegen nach 24 Stunden bestimmt. Die erhaltenen Werte zeigen die synergistische Wirkung der verwendeten erfindungsgemässen Verbindung 1-(2,4-Dichlor-phenylimino-cyclopropylmethyl)-1H-1,2,4-triazol nachstehend als "Synergist" bezeichnet, in Bezug auf das bekannte Insektizid "Nuvacron" [O,O-Dimethyl-O-(2-methylcarbamoyl-1-methylvinyl)-phosphat]:

| applizierte Menge der verwendeten Wirkstoffe pro Versuchstier (Musca domestica) | prozentuale Mortalität |
|---|---|
| 5 µg "Nuvacron" | 20 % |
| 5 µg "Synergist" | < 10 % |
| 5 µg "Nuvacron" + 5 µg "Synergist" | 100 % |
| 10 µg "Nuvacron" | 24 % |
| 10 µg "Synergist" | 15 % |
| 10 µg "Nuvacron" + 10 µg "Synergist" | 100 % |

b) Mit Hilfe eines Mikro-Applikators wurden auf Larven von Spodoptere littoralis im vierten Larvalstadium, die gegen insektizide Phosphorsäureester resistent waren, pro Versuchstier 2 µl der jeweiligen Wirkstofflösung (Lösungsmittel Aceton/Wasser : 80/20) topikal appliziert.

Die prozentuale Mortalität der Versuchstiere wurde nach 24 Stunden bestimmt. Die erhaltenen Werte zeigen die synergistische Wirkung der verwendeten erfindungsgemässen Verbindung 1-(2,4-Dichlorphenylimino-cyclopropylmethyl)-1H-1,2,4-triazol, nachstehend als "Synergist" bezeichnet, in Bezug auf das bekannte Insektizid "Nuvacron" [O,O-Dimethyl-O-(2-methylcarbamoyl-1-methylvinyl-phosphat]:

| applizierte Menge der verwendeten Wirkstoffe pro Versuchstier (Spodoptera littoralis Larven) | prozentuale Mortalität |
|---|---|
| 4 µg "Nuvacron" | 10 % |
| 4 µg "Synergist" | 8 % |
| 4 µg "Nuvacron" + 4 µg "Synergist" | 93 % |

Patentansprüche

1. Verbindung der Formel I

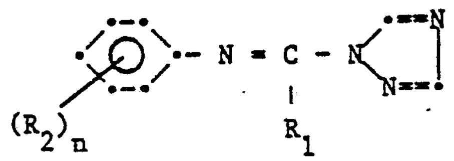

(I)

worin n für eine ganze Zahl zwischen 0 und 4 steht, $R_1$ $C_1$-$C_6$-Halogenalkyl, $C_3$-$C_6$ Cycloalkyl oder $C_3$-$C_6$ Halogencycloalkyl bedeutet, und $R_2$ eine durch n bestimmte Zahl gleicher oder verschiedener Substituenten aus der Gruppe Halogen, $C_1$-$C_4$-Alkyl, $C_1$-$C_4$-Alkoxy, Nitro, Trifluormethyl oder Cyano bedeutet.

2. Verbindung der Formel I gemäss Anspruch 1, dadurch gekennzeichnet, dass

$R_1$ durch 1-3 Halogenatome substituiertes Methyl, $C_3$-$C_6$-Cycloalkyl oder durch 1 Chloratom substituiertes $C_3$-$C_6$ Cycloalkyl und

$R_2$ Fluor, Chlor, Brom, Methoxy, Methyl, Aethyl oder Trifluormethyl bedeuten.

3. Verbindung der Formel I gemäss Anspruch 2, dadurch gekennzeichnet, dass

$R_1$ $C_3$-$C_6$-Cycloalkyl oder durch 1 Chloratom substituiertes Cyclohexyl und

$R_2$ Fluor, Chlor, Methoxy oder Trifluormethyl bedeuten.

4. Verbindung der Formel I gemäss Anspruch 3, dadurch gekennzeichnet, dass

$R_1$ $C_3$-$C_6$-Cycloalkyl und

$R_2$ Chlor

bedeuten.

5. Verbindung der Formel I gemäss einem der Ansprüche 1 bis 4, dadurch gekennzeichnet, dass n für eine ganze Zahl zwischen 0 und 2, vorzugsweise für 1 oder 2, steht.

6. Verbindung der Formel I gemäss Anspruch 4, dadurch gekennzeichnet, dass

n für 2 steht,

$R_1$ Cyclopropyl und

$R_2$ Chlor bedeuten.

7. Verbindung gemäss Anspruch 6 der Formel

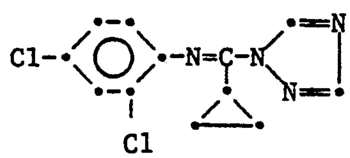

.

8. Verbindung gemäss Anspruch 2 der Formel

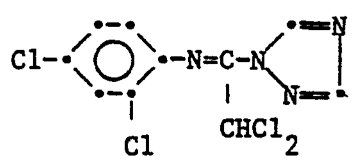

.

9. Verfahren zur Herstellung einer Verbindung der Formel I gemäss Anspruch 1, dadurch gekennzeichnet, dass man eine Verbindung der Formel II

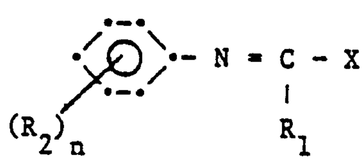

(II)

mit einer Verbindung der Formel III

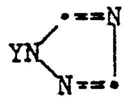

(III)

umsetzt, wobei in den Formeln II und III $R_1$, $R_2$ und n die für Formel I angegebenen Bedeutungen haben, X für ein Nukleofug und Y für Wasserstoff oder ein Metallatom stehen.

10. Schädlingsbekämpfungsmittel enthaltend als aktive Komponente mindestens eine Verbindung gemäss Anspruch 1 bis 8.

11. Mittel gemäss Anspruch 10 zur Bekämpfung von phytopathogenen Mikroorganismen und Vertretern der Ordnung Akarina.

12. Mittel gemäss Anspruch 10, welches als synergistisch aktive Komponente eine Verbindung gemäss den Ansprüchen 1 bis 8 zusammen mit einem pestizid wirksamen Phosphorsäureester, Chlorkohlenwasserstoff, Carbamat oder Pyrethroid enthält.

13. Mittel gemäss Anspruch 12 zur Bekämpfung von Insekten, welches als synergistisch aktive Komponente eine Verbindung gemäss den Ansprüchen 1 bis 8 zusammen mit einem pestizid wirksamen Phosphorsäureester enthält.

14. Mittel gemäss Anspruch 13, welches als synergistisch aktive Komponente die Verbindung gemäss Anspruch 7 enthält.

15. Verwendung einer Verbindung gemäss den Ansprüchen 1 bis 8 zur Bekämpfung von Schädlingen.

Europäisches Patentamt

# EUROPÄISCHER RECHERCHENBERICHT

Nummer der Anmeldung

EP 80 81 0126

| EINSCHLÄGIGE DOKUMENTE | | | KLASSIFIKATION DER ANMELDUNG (Int.Cl. 3) |
|---|---|---|---|
| Kategorie | Kennzeichnung des Dokuments mit Angabe, soweit erforderlich, der maßgeblichen Teile | betrifft Anspruch | |
| | DE - A - 2 321 330 (BAYER)<br>* Patentansprüche *<br>---- | 1,9,10,15 | C 07 D 249/08<br>A 01 N 43/64//<br>C 07 C 119/00 |
| | | | RECHERCHIERTE SACHGEBIETE (Int. Cl. 3) |
| | | | C 07 D 249/08 |
| | | | KATEGORIE DER GENANNTEN DOKUMENTE<br>X: von besonderer Bedeutung<br>A: technologischer Hintergrund<br>O: nichtschriftliche Offenbarung<br>P: Zwischenliteratur<br>T: der Erfindung zugrunde liegende Theorien oder Grundsätze<br>E: kollidierende Anmeldung<br>D: in der Anmeldung angeführtes Dokument<br>L: aus andern Gründen angeführtes Dokument<br>&: Mitglied der gleichen Patentfamilie, übereinstimmendes Dokument |
| X | Der vorliegende Recherchenbericht wurde für alle Patentansprüche erstellt. | | |

| Recherchenort | Abschlußdatum der Recherche | Prüfer |
|---|---|---|
| Den Haag | 27.06.1980 | CREMERS |